# FASCICULE DE BREVET EUROPEEN

(11) **EP 1 811 953 B1**
(45) Date de publication et mention de la délivrance du brevet: **16.12.2009**
(21) Numéro de dépôt: 05812046.0
(22) Date de dépôt: 21.10.2005
(51) Int. Cl.: A61K 8/92, A61K 8/63, A61K 8/49, A61K 8/36, A61Q 19/06

(54) **NOUVELLE UTILISATION D'HUILE DE CHAULMOOGRA ET DE GUGGULIPIDES EN THERAPEUTIQUE ET EN COSMETIQUE.**
NEUARTIGE VERWENDUNG VON CHAULMOOGRA-ÖL UND GUGGULIPIDEN IN THERAPEUTIKA UND KOSMETIKA
NOVEL USE OF CHAULMOOGRA OIL AND GUGGULIPIDS IN THERAPEUTICS AND COSMETICS

(30) Priorité: 22.10.2004 FR 0411242; 22.10.2004 FR 0411241; 25.10.2004 FR 0411350
(43) Date de publication de la demande: 01.08.2007
(73) Titulaire: Laboratoire Nuxe, 75010 Paris (FR)
(72) Inventeur: LECLERE, Jacques, F-45500 SAINT-GONDON (FR); FIORENTINO, Julia, F-75013 PARIS (FR)
(74) Mandataire: L'Helgoualch, Jean
(86) Numéro de dépôt international: PCT/FR2005/002621
(87) Numéro de publication internationale: WO 2006/045932

(56) Documents cités:
- EP-A- 0 997 149
- EP-A- 1 238 590
- WO-A-01/05356
- WO-A-2004/069262
- DE-A1- 19 605 089
- FR-A- 2 490 492
- FR-A- 2 518 402
- FR-A- 2 663 848
- FR-A- 2 706 304
- FR-A- 2 758 085
- FR-A- 2 827 774
- FR-A- 2 838 054
- US-A- 5 667 793
- US-A- 5 690 948
- US-A- 6 120 779
- US-A1- 2004 253 327
- US-A1- 2005 025 844
- US-B1- 6 277 396
- SATYAVATI G V: "GUGGULIPID: A PROMISING HYPOLIPIDAEMIC AGENT FROM GUM GUGGUL (COMMIPHORA WIGHTII)" ECONOMIC AND MEDICINAL PLANT RESEARCH, vol. 5, 1991, pages 47-82, XP001039709
- AGRAWAL H ET AL: "HPTLC method for guggulsterone - I. Quantitative determination of E- and Z-guggulsterone in herbal extract and pharmaceutical dosage form" JOURNAL OF PHARMACEUTICAL AND BIOMEDICAL ANALYSIS, NEW YORK, NY, US, vol. 36, no. 1, 21 septembre 2004 (2004-09-21), pages 33-41, XP004553432 ISSN: 0731-7085
- JAIN A P ET AL: "STUDY OF LONG TERM EFFECTS OF OLEO-RESIN OF COMMIPHORA MUKUL (GUGGUL) ON SERUM LIPIDS AND BODY WEIGHT" CLINICIAN, G.D. SEARLE, CHICAGO, IL, US, vol. 51, no. 8, 1987, pages 664-668, XP000872087 ISSN: 0090-0516
- DATABASE WPI Section Ch, Week 199118 Derwent Publications Ltd., London, GB; Class B04, AN 1991-128483 XP002335908 & JP 03 066623 A (AAMU KK) 22 mars 1991 (1991-03-22)

## Description

La présente invention concerne une nouvelle utilisation de l'huile de chaulmoogra éventuellement en combinaison avec des guggulipides en thérapeutique et en cosmétique, ainsi que des compositions comprenant de l'huile de chaulmoogra et éventuellement des guggulipides, en combinaison avec une ou plusieurs bases xanthiques, plus particulièrement pour le traitement des surcharges adipeuses et de la cellulite.

La peau comprend des couches superficielles, à savoir l'épiderme, et des couches plus profondes, le derme et l'hypoderme, et chacune possède des propriétés spécifiques permettant à l'ensemble de réagir et s'adapter aux conditions de son environnement. L'épiderme, qui est composé de trois types de cellules, à savoir des kératinocytes (90% des cellules épidermiques), des mélanocytes (2 à 3% des cellules épidermiques) et des cellules de Langerhans, constitue la couche externe et joue un rôle fondamental pour assurer la protection et le maintien d'une bonne trophicité. Le derme sert de support à l'épiderme et est principalement constitué de fibroblastes et d'une matrice extracellulaire essentiellement à base de collagène et d'élastine. Les fibres de collagène contribuent à la texture et la tonicité de la peau et l'élastine est responsable de son élasticité. D'autres cellules, comme les macrophages et les leucocytes, sont également présentes dans la couche du derme. L'hypoderme, qui est la couche la plus profonde de la peau, contient les adipocytes qui produisent des lipides pour que le tissu sous-cutané fabrique une couche grasse protégeant les muscles, les os et les organes internes contre les chocs.

Les adipocytes synthétisent des triglycérides par lipogénèse à partir d'acides gras libres et de glycérol provenant de la dégradation du glucose. Les acides gras et le glucose sont apportés à l'organisme par les aliments. Inversement, les triglycérides contenus dans les adipocytes, subissent une lipolyse sous l'action d'enzymes et libèrent du glycérol ou des esters de glycérol ainsi que des acides gras qui peuvent à leur tour circuler dans l'organisme et/ou être captés par des adipocytes où ils sont à nouveau transformés en triglycérides par lipogénèse. Ainsi, en cas de déséquilibre entre la lipogénèse et la lipolyse, il peut se produire une accumulation excessive de triglycérides qui se traduit par des surcharges adipeuses.

Les surcharges adipeuses sont des défauts susceptibles de nuire à l'aspect esthétique de l'individu, et elles constituent aussi des états physiologiques anormaux chez l'homme, mais surtout chez la femme, où les accumulations de cellulite sont toujours jugées disgracieuses. Ces états pathologiques, bien que différents de l'obésité, nécessitent généralement un traitement systémique. Bien souvent, à défaut de traitement thérapeutique, un traitement cosmétique est souhaité pour lutter contre les surcharges adipeuses, et pour en atténuer les effets disgracieux.

Aussi, il existe un besoin constant de mettre au point des compositions susceptibles de procurer un effet lipolytique utile dans le traitement des surcharges adipeuses, tant sur le plan cosmétique que thérapeutique. L'effet lipolytique d'une substance peut s'évaluer par sa capacité à déstocker les lipides adipocytaires en augmentant la concentration intercellulaire d'AMP cyclique (AMPc). Cette augmentation est dépendante de la saturation des récepteurs du neuropeptide Y et des récepteurs α2-adrénergiques. En effet, lorsque le neuropeptide Y ou les prostanoïdes se fixent sur leurs récepteurs, ils induisent une diminution de l'AMPc, qui entraîne une diminution de l'activité des lipases, et une augmentation de l'accumulation des lipides adipocytaires.

De nombreuses substances, notamment d'origine végétale, ont été proposées pour tenter de lutter contre la cellulite et les surcharges adipeuses et diverses compositions ont été mises au point. Elles peuvent être administrables par voie interne, par exemple par voie orale ou par injection, ou plus généralement par voie externe, notamment par application topique.

Les composés exerçant une certaine action par application topique se répartissent entre ceux qui ont une action de drainage circulatoire et ceux qui agissent sur l'adipocyte. Ainsi, des oligomères procyanidoliques, dérivés des pépins de raisin, des flavonoïdes, tel que le ruscus, les extraits de vigne rouge, de marron d'Inde, d'hamamélis, ont une certaine action de drainage circulatoire. La caféine, et divers dérivés de la caféine, ont une action sur les récepteurs α2-adrénergiques des membranes adipocytaires. En particulier, la caféine peut contribuer à diminuer l'oedème et la rétention d'eau, à augmenter la lipolyse et à limiter le stockage des graisses. Toutefois, la caféine à concentration élevée peut entraîner des effets secondaires, par exemple des palpitations, chez certains sujets. C'est pourquoi des dérivés ou des associations à base de caféine ont été proposés, et par exemple des caféine carboxylates comme dans le brevet FR 2.639.541 et l'association de caféine et d'extraits d'algues comme dans le brevet FR 2.490.492.

L'huile de chaulmoogra a longtemps été utilisée en médecine traditionnelle en Asie, notamment en Inde et en Chine, pour le traitement de la lèpre, avant l'apparition de médicaments à base de sulfones, notamment la diaminodiphényl-sulfone, et des antibiotiques antituberculeux tels que la rifampicine et la rifamycine. Il a été démontré que l'acide hydnocarpique, l'un des principaux constituants de l'huile de chaulmoogra, présente une activité antimycobactérienne en inhibant la multiplication de certaines mycobactéries telles que Mycobacterium leprae, comme indiqué par P.L. Jacobsen et L. Levy, Antimycobacterial Agents and Chemotherapy (1973) pp. 373-379.

Plus récemment, leur utilisation comme composant de compositions cosmétiques a été décrite, par exemple dans le brevet FR 2.706.304 relatif à des compositions destinées à harmoniser la pigmentation de la peau, grâce aux effets de pigmentation des zones cutanées achromiques, c'est-à-dire peu ou pas pigmentées, par migration de la pigmentation à partir de zones pigmentées. Le brevet FR 2.518.402 décrit une composition contenant de l'huile de chaulmoogra utilisable en cosmétique pour la normalisation des sécrétions sébacées et de la flore microbienne cutanée. L'utilisation des huiles de chaulmoogra dans des lotions capillaires destinées à favoriser la repousse des cheveux a aussi été décrite dans le brevet BE 570.171.

Les huiles de chaulmoogra, sont essentiellement extraites des graines de plantes ligneuses des régions tropicales appartenant à la famille des Flacourtiacées, notamment d'un arbre de variétés telles que Hydnocarpus wightiana et Taraktogenos kurzii. Ces plantes sont essentiellement d'origine asiatique, notamment d'Inde, du Viet-nam et des Philippines, ainsi que d'Afrique centrale et d'Amérique du sud, notamment du Brésil.

Les graines d'où sont extraites les huiles de chaulmoogra contiennent une forte proportion de lipides, comprise entre 30 et 50% selon les espèces, 15 à 20% de protides et 4 à 6% de matières minérales, ainsi que 1 à 3% d'insaponifiables, des glycérides d'acides gras insaturés à cycle penténique, constitués essentiellement par l'acide chaulmoogrique, l'acide hydnocarpique et l'acide gorlique. Il semble que ces acides soient responsables de l'action antimycobactérienne utile en thérapeutique dans le traitement traditionnel de la lèpre. On a observé que la structure spatiale de ces acides se rapproche du noyau cyclopentanoperhydroxyphénantrène caractéristique des stérols.

Les acides chaulmoogrique, hydnocarpique et gorlique peuvent être représentés par les formules générales suivantes, respectivement :

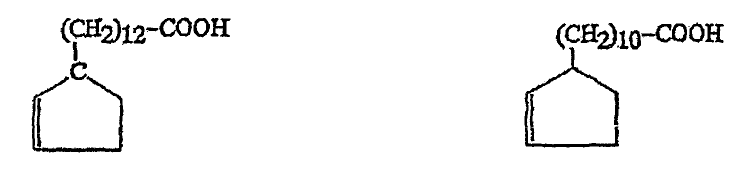

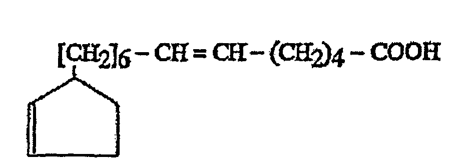

Ainsi, ces trois acides comprennent le même cycle cyclopenténique portant un groupe acide à l'extrémité d'une chaîne carbonée -(CH₂)ₙ- où n est 10 dans le cas de l'acide hydnocarpique et 12 dans le cas de l'acide chaulmoogrique, cette même chaîne comportant une double liaison dans le cas de l'acide gorlique. Les teneurs en chacun de ces trois acides dans les huiles de chaulmoogra dépendent de l'origine des espèces.

Des esters de ces acides, en particulier des esters méthyliques, éthyliques et benzyliques, ont été synthétisés en vue d'en améliorer l'acceptabilité et la tolérance. De même on a constaté que des sels tels que le chaulmoograte de sodium et l'hydnocarpate de sodium, exercent une action dissolvante sur les lécithines et le cholestérol, ce qui pourrait expliquer leur action sur l'enveloppe cireuse des bacilles de la lèpre et de la tuberculose.

On trouve également dans les huiles de chaulmoogra des acides gras du type palmitique, oléique, palmitoléique, stéarique, myristique, et des traces d'acide aleprique et aleprilique.

Les guggulipides sont extraits de la plante *Commiphora mukul* (guggul) de la famille des burséracées, qui est un arbuste à branches épineuses originaire des régions arides de l'Inde, du Pakistan et du Bangladesh. Cette plante est très utilisée depuis de nombreuses années en médecine ayurvédique dans diverses applications. L'intérêt du guggul provient de l'exsudat résineux qui est extrait de l'écorce après incision, puis séché et fragmenté, et contient des guggulipides présentant une activité anti-inflammatoire, hypocholestérolémiante et hypolipémiante, utile pour le traitement de l'obésité, de l'hypocholestérolémie et de l'arthrite en administration par voie interne. Plus récemment, le brevet EP 766.510 décrit l'utilisation d'un extrait lipophile de la résine de Commiphora mukul en application topique pour la pigmentation de la peau et des cheveux, et le brevet EP 1.238.590 décrit un additif alimentaire à base d'extrait de guggul désodorisé.

Les guggulipides sont généralement obtenus par extraction à l'acétate d'éthyle de la gomme-résine (exsudat) provenant de l'écorce de *Commiphora mukul* (guggul), préalablement séchée et broyée. L'activité hypolipidémique des guggulipides est principalement due à la présence de deux isomères dérivés du pregnane, la Z-guggulstérone et la E-guggulstérone, qui ont pour effet de diminuer les taux de LDL et VLDL-cholestérol et d'augmenter le taux de HDL-cholestérol. Ces isomères sont représentés par les formules générales suivantes.

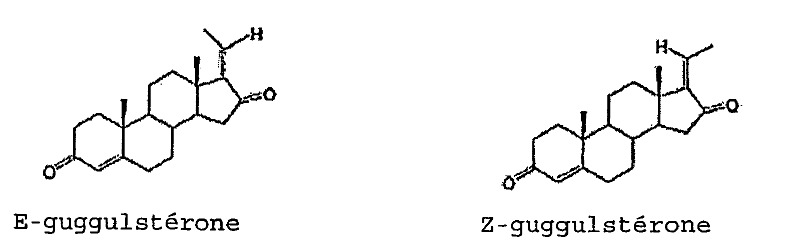

Les structures et propriétés de ces dérivés sont décrites notamment par I. Kimura et al., Bioorganic Med. Chem. Letters, vol. 11 (8) (23/04/2001) pp. 985-989 et par J. Wu et al., Molecular Endocrinology, 16 (7) pp. 1590-1597 (2002).

Les études réalisées par la demanderesse sur les récepteurs cellulaires et la protection des cellules dendritiques ont maintenant démontré de manière inattendue que l'huile de chaulmoogra et les guggulipides présentent un effet lipolytique vérifié par leur action sur les adipocytes humains en culture, se traduisant par la compétition de l'huile de chaulmoogra et des extraits de guggulipides, appliqués par voie externe, vis-à-vis des récepteurs du neuropeptide Y et α2 adrénergiques.

La présente invention a donc pour objet une nouvelle utilisation de l'huile de chaulmoogra et/ou de ses composants, éventuellement en combinaison avec des guggulipides et/ou de la Z-guggulstérone et de la E-guggulstérone en une concentration appropriée, pour préparer des compositions cosmétiques destinées au traitement et à la prévention des surcharges adipeuses et de la cellulite, ainsi qu'une nouvelle utilisation de l'huile de chaulmoogra et/ou de ses composants éventuellement en combinaison avec des guggulipides et/ou de la Z-guggulstérone et de la E-guggulstérone en une concentration appropriée, pour préparer des compositions pharmaceutiques destinées au traitement et à la prévention des surcharges adipeuses et de la cellulite

Les composants de l'huile de chaulmoogra sont de préférence les acides chaulmoogrique, hydnocarpique et gorlique ainsi que leurs sels et esters, qui peuvent être choisis parmi les esters de méthyle, d'éthyle ou de benzyle, ainsi que les sels de sodium ou de potassium.

La teneur en huile de chaulmoogra, ou en acides ou sels ou esters des compositions suivant la présente invention est généralement comprise entre 0,1 et 20%, et de préférence entre 1 et 10% en poids par rapport au poids total de la composition, pour procurer les meilleurs effets lipolytiques.

La teneur en guggulipides des compositions suivant la présente invention est généralement comprise entre 0,05 et 20%, et de préférence entre 1 et 10% en poids par rapport au poids total de la composition, pour procurer les meilleurs effets lipolytiques.

L'invention a également pour objet l'utilisation de l'huile de chaulmoogra, ou de ses composants essentiels tels que les acides chaulmoogrique, hydnocarpique et gorlique, ainsi que leurs sels et esters, éventuellement en combinaison avec des guggulipides et/ou de la Z-guggulstérone et de la E-guggulstérone pour la préparation d'une composition cosmétique pour le traitement des surcharges adipeuses et de la cellulite.

L'invention porte également sur l'utilisation de l'huile de chaulmoogra, ou de ses composants essentiels tels que les acides chaulmoogrique, hydnocarpique et gorlique, ainsi que leurs sels et esters, éventuellement en combinaison avec des guggulipides et/ou de la Z-guggulstérone et de la E-guggulstérone en tant qu'agent de traitement des surcharges adipeuses et de la cellulite pour la préparation d'une composition cosmétique.

L'invention a aussi pour objet l'utilisation de l'huile de chaulmoogra, et/ou de ses composants essentiels tels que les acides chaulmoogrique, hydnocarpique et gorlique, ainsi que leurs sels et esters, éventuellement en combinaison avec des guggulipides et/ou de la Z-guggulstérone et de la E-guggulstérone pour la préparation d'un médicament à effet lipolytique pour le traitement et la prévention des surcharges adipeuses et de la cellulite.

L'invention a encore pour objet un procédé de traitement cosmétique de la peau, et plus particulièrement pour prévenir ou réduire les surcharges adipeuses et la cellulite, par application d'une composition à base d'huile de chaulmoogra éventuellement en combinaison avec des guggulipides et/ou de la Z-guggulstérone et de la E-guggulstérone sur la zone de la peau nécessitant un tel traitement.

Les expérimentations effectuées ont montré que l'activité lipolytique de l'huile de chaulmoogra était essentiellement due aux acides chaulmoogrique, hydnocarpique et gorlique qu'elle contient, et que l'activité lipolytique des guggulipides était essentiellement due à la Z-guggulstéronè et à la E-guggulstérone qu'ils contiennent.

Plus particulièrement, il a été démontré que
- une composition à base d'huile de chaulmoogra éventuellement en combinaison avec des guggulipides procure une protection vis-à-vis de l'inhibition du TNFα (facteur onconécrosant) dès la concentration de 0,5 à 1%, ce qui laisse supposer une action sur les prostanoïdes, et par conséquent sur la lipogénèse.
- les prostanoides tels que la prostaglandine E2 sont des molécules qui stimulent la lipogénèse après fixation sur les récepteurs des adipocytes, et cette stimulation est médiée par la phospholipase C. Aussi, comme indiqué plus loin, l'effet lipolytique de l'huile de chaulmoogra et des guggulipides est démontré par leur effet de blocage de la fixation des prostanoides sur leur récepteur par compétition avec eux.
- le neuropeptide Y est un neurotransmetteur qui stimule la pénétration des glucides par les adipocytes après fixation sur son récepteur, et cette accumulation de glucides par les cellules des tissus adipeux augmente leur capacité à synthétiser et à stocker les lipides. Or, les études effectuées par la demanderesse ont montré que l'huile de chaulmoogra et les guggulipides entrent en compétition avec le récepteur du neuropeptide Y et possèdent donc une activité lipolytique.

Les huiles de chaulmoogra utilisées dans la présente invention peuvent être extraites des graines de plantes de variétés telles que Hydnocarpus wightiana, Taraktogenos kurzii, Hydnocarpus alpina, Hydnocarpus anthelmintica, Hydnocarpus cauliflora, Hydnocarpus dawnensis, Hydnocarpus heterophylla, Hydnocarpus hutchinsonii, Hydnocarpus ovoidea, Hydnocarpus subfalcata, Hydnocarpus venenata, Hydnocarpus verrucosa, Hydnocarpus woodii, Hydnocarpus calvipetala, Hydnocarpus ilicifolia, Hydnocarpus octandra, Gynocardia odorata, Oncoba echinata, Caloncoba glauca, Caloncoba welwitschii, Carpotroche brasiliensis, Carpotroche amazonica, Asteriastigma macrocarpa, Mayna odorata et Lindakeria dentata.

Les guggulipides utilisés dans la présente invention peuvent être obtenus par extraction à l'acétate d'éthyle de la gomme-résine provenant de l'écorce de *Commiphora mukul,* séchée et broyée, suivant les techniques usuelles d'extraction.

L'invention porte en outre sur une composition cosmétique et/ou pharmaceutique destinée au traitement et à la prévention des surcharges adipeuses et de la cellulite, comprenant de l'huile de chaulmoogra et/ou au moins un de ses composants, tels que l'acide chaulmoogrique, hydnocarpique et gorlique ainsi que leurs sels et esters, et éventuellement des guggulipides et/ou de la Z-guggulstérone et de la E-guggulstérone, en combinaison avec une ou plusieurs bases xanthiques, notamment la caféine.

Les sels ou esters des acides chaulmoogrique, hydnocarpique et gorlique peuvent être choisis parmi les esters de méthyle, d'éthyle ou de benzyle, ainsi que les sels de sodium ou de potassium, et par exemple le chaulmoograte de sodium ou de potassium et l'hydnocarpate de sodium.

Les bases xanthiques sont constituées par les trois bases puriques, à savoir la caféine, la théophylline et la théobromine, isolément ou en association, et il s'agit de préférence de la caféine.

La xanthine est un composé dérivé de la purine, comportant deux cycles accolés, pyridinyle et glyoxalinyle, constituant une dioxy-2,6 purine. La xanthine peut être obtenue par réduction de l'acide urique. La théobromine, la théophylline et la caféine sont des dérivés méthylés de la xanthine qui diffèrent entre eux par la position et le nombre de groupements méthyles. La théobromine, ou diméthyl-1,3 xanthine, est un alcaloïde extrait de la graine de cacaoyer, *Theobroma cacao ;* on en trouve aussi en faible quantité dans le thé et le café. La théophylline, ou diméthyl-1, 3 xanthine, est un alcaloïde extrait des feuilles du théier, *Thea sinensis.* On peut aussi en faire la synthèse par méthylation de la xanthine. La caféine, ou triméthyl-1,3,7 xanthine, est un alcaloïde extrait des feuilles du théier, et des graines de café. La caféine peut également être obtenue à partir des graines du kolatier, du guarana et du maté, ainsi qu'à partir des graines de porangaba *(Cordia salicifolia)* de la famille des boraginées. On peut aussi l'obtenir par synthèse en méthylant la théobromine et la théophylline.

Les études réalisées par la demanderesse sur les récepteurs cellulaires et la protection des cellules dendritiques ont démontré de façon inattendue que l'effet lipolytique de l'huile de chaulmoogra, éventuellement en combinaison avec des guggulipides, évoqué plus haut pouvait potentialiser celui des bases xanthiques, en particulier de la caféine.

Ainsi, il a été démontré notamment qu'en utilisant une composition comprenant 0,5% d'huile de chaulmoogra et 0,4% de caféine, la proportion de glycérol libéré dans le milieu de culture, lors des tests d'évaluation de l'effet lipolytique au niveau des adipocytes, est de 40% alors qu'elle n'est que de 30% avec la caféine seule.

### Préparation des adipocytes humains en culture

L'étude a été réalisée sur des adipocytes humains en culture, par la méthode de la liaison du neuropeptide Y et des prostanoïdes sur leurs récepteurs au niveau de la cellule adipocytaire.

Les adipocytes sont obtenus par prélèvement du tissu adipeux à partir d'une chirurgie plastique (peau entière), conservé à température ambiante dans un milieu de conservation approprié puis découpé finement et placé dans un tampon HBSS à pH 7,4. Une solution de collagénase de type II (1 mg/ml dans le tampon HBSS à pH 7,4) est ajoutée au tube contenant les fragments de tissu adipeux (4 volumes de collagénase pour 1 volume de tissu) et l'ensemble est incubé à 37°C pendant 40 à 60 minutes sous agitation constante, sans dioxyde de carbone. Cette première étape permet de libérer les adipocytes de la gangue conjonctive et du stroma vasculaire.

Après incubation, les cellules sont récupérées par filtration sur un filtre en nylon et les adipocytes ainsi isolés sont séparés par flottation. Après élimination du sous-nageant par aspiration, la suspension adipocytaire est lavée par un tampon isotonique KBR pour éliminer toute trace de collagénase. Après addition de 3,5% de BSA, le pH est ajusté à 7,5 et le tampon est filtré (filtre 0,22 *µ*m).

### Compétition vis-à-vis des récepteurs α-2 adrénergiques

Les prostanoïdes comme la prostaglandine E2 sont des molécules qui stimulent la lipogénèse après fixation sur les récepteurs des adipocytes. Une méthode pour démontrer l'effet lipolytique d'une substance consiste donc à bloquer la fixation des prostanoïdes sur leur récepteur par compétition avec eux.

L'effet de compétition de l'huile de chaulmoogra et des guggulipides de l'invention vis-à-vis des récepteurs α-2 adrénergiques est vérifié comme indiqué ci-après.

Les adipocytes humains en culture sont répartis en plusieurs lots incubés pendant 1 heure sous agitation. La réaction de liaison est réalisée en présence de ^{H}C-PGE2 (43,8 Ci/mmol). Les lots n° 1 et 1' sont des témoins négatifs ne contenant pas d'huile de chaulmoogra ni de guggulipides et sans traitement. Les lots n° 2 et 2' sont des témoins positifs ne contenant pas d'huile de chaulmoogra ni de guggulipides et soumis au traitement. Les lots n° 3 et 3', n° 4 et 4' et n° 5 et 5' contiennent respectivement 0,5%, 1% et 5% en poids d'huile de chaulmoogra (lots 3, 4 et 5) et 0,3%, 0,5% et 1% en poids de guggulipides (lots 3', 4' et 5') suivant l'invention.

Le tableau A des résultats indiqués ci-dessous montre que l'huile de chaulmoogra entre en compétition avec la PGE2 et bloque la fixation sur les récepteurs des adipocytes dès la concentration de 0,5% en poids.

**Tableau A**

| | Cpm ± écart type | Variation % |
|---|---|---|
| Témoin (lot n° 2) | 10529 ± 145 | |
| Huile de chaulmoogra à 0, 5% (lot 3) | 7817 ± 229 | - 25 |
| Huile de chaulmoogra à 1% (lot 4) | 5118 ± 303 | - 51 |
| Huile de chaulmoogra à 5% (lot 5) | 5007 ± 196 | - 52 |

Les résultats sont exprimés en coups par minute (Cpm).

De même, le tableau B des résultats indiqués ci-dessous montre que les guggulipides entrent en compétition avec la PGE2 et bloquent la fixation sur les récepteurs des adipocytes, de manière significative, dès la concentration de 0,5% en poids.

**Tableau B**

| | Cpm ± écart type | Variation % |
|---|---|---|
| Témoin (lot n° 2') | 12002 ± 615 | |
| Guggulipides à 0,3% (lot 3') | 11410 ± 704 | - 5 (ns) |
| Guggulipides à 0,5% (lot 4') | 9726 ± 481 | - 19 |
| Guggulipides à 1% (lot 5') | 9453 ± 803 | - 21 |

| | | |
|---|---|---|
| ns = non significatif | | |

### Compétition vis-à-vis du récepteur du neuropeptide Y

L'effet de compétition de l'huile de chaulmoogra et des guggulipides de l'invention vis-à-vis du récepteur du neuropeptide Y est vérifié comme indiqué ci-après.

Les adipocytes sont répartis en plusieurs lots comme indiqué ci-dessus, mais la réaction de liaison est réalisée en présence de ¹²⁵I-NY (73,3 Ci/mmol).

On sait que le neuropeptide Y est un neurotransmetteur qui stimule la pénétration des glucides par les adipocytes après fixation sur son récepteur. Les résultats indiqués aux tableaux C et D ci-dessous montrent respectivement que l'huile de chaulmoogra et les guggulipides entrent en compétition avec le neuropeptide Y et bloquent sa fixation sur son récepteur dès la concentration de 0,5% en poids, en limitant sa capacité à synthétiser et stocker des lipides.

**Tableau C**

| | Cpm ± écart type | Variation % |
|---|---|---|
| Témoin (lot n° 2) | 7280 ± 315 | |
| Huile de chaulmoogra à 0,5% (lot 3) | 6412 ± 251 | - 12 |
| Huile de chaulmoogra à 1% (lot 4) | 5032 ± 111 | - 30 |
| Huile de chaulmoogra à 5% (lot 5) | 4805 ± 98 | - 33 |

**Tableau D**

| | Cpm ± écart type | Variation % |
|---|---|---|
| Témoin (lot n° 2') | 8321 ± 415 | |
| Guggulipides à 0,3% (lot 3') | 7852 ± 523 | - 6 (ns) |
| Guggulipides à 0,5% (lot 4') | 7232 ± 385 | - 13 |
| Guggulipides à 1% (lot 5') | 6805 ± 293 | - 18 |

Ces résultats mettent en évidence l'effet lipolytique des huiles de chaulmoogra et des guggulipides de l'invention, dès la concentration de 0,5% en poids.

### Dosage enzymatique du glycérol

L'efficacité des guggulipides et de l'association de bases xanthiques et d'huile de chaulmoogra suivant la présente invention a aussi été vérifiée sur des tests d'évaluation de l'effet lipolytique par relargage du glycérol dans un milieu de culture d'adipocytes humains. Cette activité est en effet significative d'un effet lipolytique se traduisant par une hydrolyse des lipides totaux en glycérol et en acides gras libres.

Le dosage enzymatique du glycérol consiste à doser l'oxydation de la forme réduite du nicotinamide-adénine-dinucléotide (NADH) par mesure de la densité optique (DO) à 385 nm. En effet, la quantité de NADH oxydée au cours de la réaction de conversion du glycérol en pyruvate puis en lactate est proportionnelle à la concentration totale du glycérol.

Sous l'action de trois enzymes (glycérokinase, pyruvate kinase et lactate déshydrokinase) les trois réactions suivantes se produisent : 1) le glycérol réagit avec l'ATP pour donner le glycérol-3-phosphate et l'ADP ; 2) l'ADP réagit avec le phosphoénolpyruvate pour libérer du pyruvate et de l'ATP ; 3) enfin, le pyruvate réagit avec la NADH-H⁻ pour procurer le lactate-NAD.

On utilise un tampon glycilglycine (pH 7,4) contenant NADH (0,83 mg), ATP (2 mg), sulfate de magnésium (1 mg) et phosphoénolpyruvate (1 mg). La suspension enzymatique est constituée de pyruvate kinase (240 u) et de lactate déshydrogénase (220 u). La glycérokinase (34 u) est utilisée en suspension.

La méthode consiste à mélanger dans un premier temps tous les éléments des réactions 2) et 3) ci-dessus, en présence et en l'absence des milieux de culture des adipocytes (avec et sans le produit à tester), et de mesurer la DO (A₁), et dans un deuxième temps à démarrer la réaction 1) en ajoutant la glycérokinase. La DO résultante est alors mesurée à 385 nm (A₂). La différence de DO est obtenue à partir des différences de DO pour le produit à tester d'une part, et pour le témoin d'autre part.

Le tableau E des résultats indiqués ci-dessous montre que les guggulipides présentent un effet lipolytique significatif aux doses testées. Les lots 1', 3', 4' et 5' sont les mêmes que dans les tests précédents. Le lot 2' est un témoin positif traité par la caféine à la concentration de 0,4%.

**Tableau E**

| | glycérol (mg/l) | augmentation % |
|---|---|---|
| Témoin négatif(lot n° 1') | 42,7 ± 3,9 | |
| Témoin positif (caféine à 0,4% - lot 2') | 56,9 ± 2,7 | + 33% |
| Guggulipides à 0,3% (lot 3') | 43,8 ± 7,5 | + 2% |
| Guggulipides à 0,5% (lot 4') | 47,3 ± 2,2 | + 10% |
| Guggulipides à 1% (lot 5') | 49,9 ± 1,3 | + 16% |

Ces résultats mettent en évidence l'effet lipolytique des guggulipides suivant l'invention, dès la concentration de 0,5% en poids.

Pour évaluer l'efficacité de l'association de bases xanthiques et d'huile de chaulmoogra, les adipocytes humains sont répartis en plusieurs lots incubés pendant 1 heure sous agitation. Le lot n°1" est un témoin négatif ne contenant pas de produit et sans traitement. Le lot n°2 " est un témoin positif soumis au traitement uniquement de la caféine à 0,4%. Les lots n°3 " , n°4" et n°5" contiennent respectivement 0,5%, 1% et 5% en poids d'huile de chaulmoogra dans le premier test (Tableau F), et les mêmes concentrations d'huile de chaulmoogra additionnée de caféine à 0,4% dans le deuxième test (Tableau G) .

Le tableau F des résultats indiqués ci-dessous montre que l'huile de chaulmoogra, utilisée isolément, présente un effet lipolytique inférieur à celui de la caféine aux doses testées.

**Tableau F**

| | glycérol (mg/l) | augmentation % |
|---|---|---|
| Témoin (lot n° 1" ) | 52, 8 ± 4,2 | |
| Témoin positif (caféine à 0, 4%) | 68,5 ± 5,5 | + 30% |
| Huile de chaulmoogra à 0, 5% (lot 3") | 55,7 ± 5,7 | + 6% |
| Huile de chaulmoogra à 1% (lot 4") | 64,8 ± 3,9 | + 23% |
| Huile de chaulmoogra à 5% (lot 5" ) | 66,7 ± 4,4 | + 27% |

Ces résultats montrent que l'effet lipolytique de l'huile de chaulmoogra, aux concentrations de 1 et 5%, est légèrement inférieur à celui de la caféine.

**Tableau G**

| | glycérol (mg/l) | augmentation % |
|---|---|---|
| Témoin (lot n°1" ) | 52,8 ± 4,2 | |
| Témoin positif (caféine à 0,4%) | 68,5 ± 5,5 | + 30% |
| Huile de chaulmoogra 0,5% + caféine 0,4% (lot 3") | 73,9 ± 6,1 | + 40% |
| Huile de chaulmoogra 1% + caféine 0, 4% (lot 4") | 69,9 ± 4,1 | + 33% - |
| Huile de chaulmoogra 5% + caféine 0,4% (lot 5") | 68,4 ±5,3 | + 30% |

Ces résultats mettent en évidence l'effet lipolytique de l'association de la caféine et de l'huile de chaulmoogra suivant l'invention, dès la concentration de 0,5% en poids d'huile de chaulmoogra pour une concentration de 0,4% de caféine. Cet effet lipolytique est particulièrement net car l'augmentation du taux de glycérol libéré dans le milieu de culture des adipocytes humains est de 40%, ce qui montre que l'effet lipolytique de la caféine a été potentialisé par la présence de l'huile de chaulmoogra.

Dans la composition selon l'invention, le rapport en poids des bases xanthiques, notamment de la caféine, à l'huile de chaulmoogra est généralement compris entre 1:0,5 et 1:5 environ et de préférence entre 1:1 et 1:1,5, et de préférence voisin de 1:1,25.

En particulier, la teneur en base xanthique, plus particulièrement en caféine, peut être comprise entre 0,1 et 8%, et de préférence entre 0, 5 et 5% en poids, tandis que la teneur en huile de chaulmoogra, ou en acides ou sels ou esters est généralement comprise entre 0,1 et 15%, et de préférence entre 1 et 8% par rapport au poids total de la composition, pour procurer les meilleurs effets lipolytiques.

Dans le cas de teneurs relativement élevées en caféine et en huile de chaulmoogra, il peut être avantageux d'utiliser un milieu comportant une phase aqueuse présentant une constante diélectrique d'environ 60, par exemple au moyen d'un milieu présentant un degré alcoolique (par exemple éthylique) d'environ 30%.

L'huile de chaulmoogra, ou ses acides ou esters, les guggulipides, et les bases xanthiques, notamment la caféine, peuvent être utilisés avantageusement sous une forme encapsulée dans des liposomes.

Suivant une technique connue dans la fabrication des compositions cosmétiques, les liposomes sont constitués par des petites sphères creuses, de diamètre généralement inférieur à 500 nm, dont la paroi est formée d'une double couche de lipides tels que des glucolipides ou des phospholipides. Ils peuvent être obtenus par exemple par traitement aux ultrasons d'un mélange d'un soluté aqueux et de lipides. Les lipides (phospholipides ou glucolipides) se réorganisent dans une configuration où l'énergie de l'ensemble est minimale, donc thermodynamiquement la plus stable. Les liposomes sont utilisés dans l'industrie cosmétique pour délivrer des composés à l'intérieur des cellules lorsque le vésicule fusionne avec la membrane plasmique.

Suivant une forme préférée de réalisation de l'invention, une partie au moins de la caféine et de l'huile de chaulmoogra est encapsulée dans des vésicules du type liposome, et le rapport en poids de la caféine à l'huile de chaulmoogra, de préférence voisin de 1,25, est assuré par une vectorisation appropriée dans les liposomes.

La composition selon l'invention peut contenir en outre un agent veinotonique, qui peut être choisi parmi les extraits végétaux à saponosides, les extraits végétaux à coumarine, et les extraits végétaux à flavonoïdes.

Les plantes à saponosides ont une activité veinotonique importante ainsi que des propriétés anti-inflammatoires et anti-oedémateuses. Elles ont aussi fréquemment des propriétés vasoconstrictives favorisant la circulation capillaire ainsi que des propriétés décongestionnantes améliorant la circulation lymphatique. Ces plantes peuvent être choisies parmi le fragon *(Ruscus aculeatus* dont on extrait la ruscogénine), le lierre (qui fournit l'hédéragénine), le marronnier d'Inde *(Aesculus hippocastanum* dont on extrait l'escine).

Les plantes à coumarine procurent une action anti-oedémateuse, vasculo-protectrice et veinotonique, et peuvent être choisies parmi le marronnier d'Inde (dont on extrait l'esculoside), et le melilot *(Melilotus officinalis,* dont on extrait le mélilotoside qui s'hydrolyse en coumarine).

Les plantes à flavonoïdes ont pour effet de diminuer la fragilité capillaire et de renforcer la résistance des vaisseaux. Elles peuvent être choisies parmi le gingko *(Gingko* biloba) et le *Sophora japonica* dont on peut extraire la troxérutine.

La composition selon l'invention peut contenir en outre un agent restructurant.

Les restructurants éventuellement ajoutés aux compositions utilisées dans la présente invention peuvent être choisis parmi les silanols, la *Centella asiatica,* l'hespéritine méthyl chalcone et les alpha-hydroxy acides.

La *Centella asiatica* est un stimulant de la synthèse du collagène et des mucopolysaccharides par les fibroblastes. De plus, il exerce une action positive sur les symptômes de l'insuffisance veineuse chronique des membres inférieurs. L'hespéritine méthyl chalcone possède une action anti-glycation et empêche la rigidification des fibres de collagène entre elles. Les AHA, ou alpha-hydroxy acides, sont connus pour améliorer la pénétration des actifs grâce à leur action kératolytique.

Les compositions conformes à la présente invention sont de préférence administrables par voie topique. Suivant la terminologie classique, l'administration par voie topique désigne toute méthode consistant à appliquer la substance ou la composition directement sur la peau, sur la zone nécessitant le traitement.

Conformément à la présente invention, la composition administrable par voie topique peut avantageusement contenir, outre les composants de base décrits ci-dessus, une ou plusieurs autres substances connues pour exercer des effets complémentaires bénéfiques pour la peau, et plus particulièrement le tocophérol, la vitamine A (rétinol), l'acide rétinoique, des agents bactéricides, de la théophylline ainsi que des extraits végétaux connus pour favoriser l'effet d'amincissement comme des extraits huileux de thé vert et de café vert, des extraits huileux de Garcinia cambodgiana ou de Chondrus crispus, ou un extrait alcoolique de lierre ou de liane du Pérou, ou des agents drainants tels que des mucopolysaccharides et par exemple des extraits de laminaria digitata.

Certains de ces extraits, notamment l'extrait glycolique de lierre (hédéragénine), présentent l'avantage de faciliter la pénétration de la composition de l'invention à travers l'épiderme et le derme. D'autres composés connus pour faciliter la pénétration des principes actifs de compositions cosmétiques à travers l'épiderme sont par exemple l'éthoxydiglycol et des saponosides du type hédéragénine.

On peut aussi ajouter avantageusement un composé accélérant la microcirculation et augmentant la température locale, tel que par exemple le nicotinate de méthyle, afin de faciliter l'élimination des glycérides.

Les compositions cosmétiques et pharmaceutiques conformes à la présente invention, sont destinées de préférence à une administration topique, et contiennent donc des supports et excipients couramment utilisés dans des compositions de ce type telles que des émulsions H/E ou E/H, des crèmes, des gels ou des lotions. Dans le cas des émulsions, la phase grasse peut représenter entre 10 et 60% environ du poids de la composition, la phase aqueuse entre 10 et 80% environ et l'agent émulsionnant entre 2 et 20%, le reste étant constitué par les composants de base indiqués ci-dessus et les autres composants mentionnés ci-après.

La composition peut encore contenir diverses substances et excipients choisis en fonction de leurs propriétés connues et de la forme galénique envisagée. Ainsi, on peut incorporer dans la composition des conservateurs, des agents émulsionnants, des agents viscosants, des épaississants, des gélifiants, des antioxydants, des agents hydratants, des tensioactifs, des parfums, des huiles, des lipides, un solvant spécifique ainsi que de l'eau et divers additifs destinés à améliorer les propriétés physiques de la composition.

On peut choisir l'agent émulsionnant parmi des polymères carboxyvinyliques à haut poids moléculaire (par exemple le Carbopol^{®}), des polysorbates (par exemple le Tween 20^{®} ou le Polysorbate 80^{®}), des esters de sorbitan et en particulier un monostéarate, un tristéarate, un monopalmitate, et un laurate de sorbitan. On peut encore utiliser d'autres agents émulsionnants tels que divers dérivés d'acide stéarique ou palmitique, et par exemple le stéarate de PEG 100®, des mono- ou diglycérides d'acide stéarique ou palmitique, un stéarate de propylène glycol auto-émulsionnable, ou encore le poly-glycéryl-2-sesquioléate, l'éther cétylique de polyoxyéthylène, un polyglucoside de siloxane, ou une silicone émulsionnable. On peut encore utiliser des mélanges émulsionnants non ioniques tels que le Protegin X^{®}.

Les agents viscosants utilisés dans les compositions de l'invention peuvent être choisis parmi divers polymères d'acide acrylique, un copolymère d'acrylate et de laurate d'acryloyle, une gomme cellulose, une silice, des polymères carboxyvinyliques, un silicate d'aluminium et de magnésium, et on peut utiliser par exemple la silice colloïdale vendue sous la marque Aerosil 200^{®} ou un acide polyacrylique réticulé tel que le Carbopol 940^{®}.

Les gélifiants ou épaississants peuvent être choisis par exemple parmi les polyacrylamides, des acrylates comme le Pemulen^{®}, les dérivés de cellulose comme l'hydroxypropyl cellulose, ou les gommes naturelles.

Les agents hydratants utilisés peuvent être choisis par exemple parmi un polyol, le sorbitol, le maltitol, le penta-érythritol, les polyacrylates et polyméthacrylates de glycéryle, le glycérol ou des dérivés de glycérol. On peut aussi ajouter des émollients tels qu'un malate d'alkyle, l'isohexadécane, des triglycérides d'acide caprique ou caprylique, etc.

Les conservateurs usuels de la technique des compositions dermatologiques ou cosmétologiques peuvent être utilisés dans l'invention, et par exemple l'acide benzoïque et un p-hydroxybenzoate d'alkyle tel que les p-hydroxy-benzoates de méthyle et de propyle (Méthylparaben et Propylparaben), un alcool tel que le phénoxy-éthanol ou encore la chlorphénésine ou l'imidazolidinyl urée.

Les constituants de la phase grasse, c'est-à-dire les huiles et lipides, peuvent être choisis parmi l'huile de jojoba, l'huile de maïs, l'huile de vaseline, l'huile de coco hydrogénée, l'huile de carthame, des glycérides d'acides gras saturés, l'acide stéarique, l'acide palmitique, le stéarate d'octyle, le palmitate de glycéryle, le palmitate d'octyle, un triglycéride d'acides caprique et caprylique, le 2-octyldodécanol, le polyéthylène glycol, l'adipate d'éthyl-2 hexyle, ou encore des huiles de silicones telles que le méthyl phényl polysiloxane, la diméthicone, la cyclométhicone, la cyclométhicone/diméthicone copolyol, la phényl diméthicone.

La composition peut aussi contenir un solvant choisi en fonction des composants utilisés et de la forme d'administration envisagée. Le solvant peut être par exemple de l'eau, et de préférence de l'eau déminéralisée, ou un solvant spécifique tel que le propylène glycol, un éther de diéthylène glycol, ou un alcool, en particulier l'éthanol.

Le pH de la composition est de préférence compris entre 5,5 et 7,5, et peut être ajusté, selon les compositions, par addition d'un acide tel que l'acide citrique ou d'une base telle que l'hydroxyde de sodium.

La composition conforme à la présente invention peut être présentée sous les formes classiquement utilisées pour une application topique, c'est-à-dire sous forme de gel, lotion, émulsion (en particulier crème ou lait), patchs transdermiques, masque ou pommade, contenant des excipients et supports usuels compatibles et pharmaceutiquement acceptables. Ces formes d'administration par voie topique sont préparées par les techniques connues, et par exemple, dans le cas d'une crème, par dispersion d'une phase grasse dans une phase aqueuse pour obtenir une émulsion huile dans eau, ou inversement pour préparer une émulsion eau dans huile. Dans le cas de crèmes, on préfère utiliser des émulsions à structure lamellaire contenant peu de produits éthoxylés ou n'en contenant pas du tout. L'huile de chaulmoogra et éventuellement les guggulipides sont de préférence préalablement chauffés avant d'être incorporés à la phase grasse, tandis que la caféine éventuellement utilisée en association est introduite dans la phase aqueuse ou alcoolique.

A titre d'exemple, on peut préparer des compositions topiques conformes à l'invention sous forme de crèmes amincissantes, de laits ou de gels amincissants, utilisables en une ou plusieurs applications quotidiennes, la durée du traitement pouvant être généralement de l'ordre de un à trois mois.

Les exemples de compositions à base d'huile de chaulmoogra et éventuellement de guggulipides, éventuellement associés à de la caféine donnés ci-après illustrent l'invention sans en limiter la portée. Sauf indication contraire, les parties et pourcentage sont indiqués en poids.

### Exemple 1

Un gel amincissant à base d'huile de chaulmoogra ayant la composition indiquée ci-après est préparé suivant les techniques usuelles :

| | | |
|---|---|---|
| huile de chaulmoogra | | 5,0 |
| EDTA trisodique | | 0,1 |
| glycérine | | 2,0 |
| octyldodécanol | | 2,0 |
| polymère carboxyvinylique | | 0,5 |
| oléate de décyle | | 2,0 |
| copolymère acrylate de sodium / laurate de | | |
| diméthyl acryloyle | | 0,5 |
| trométhamine | | 0,6 |
| phényl diméthicone | | 1,0 |
| extrait de laminaria digitata | | 7,0 |
| extrait de liane du Pérou | | 3,0 |
| parfum | | 0, 1 |
| conservateurs | | 0,6 |
| Eau déminéralisée | q.s.p. | 100,0 |

Ce gel amincissant est utilisé en application topique, une fois par jour sur les zones de la peau à traiter, pendant une période de 8 semaines. Les premiers effets peuvent être observés dès la fin de la 2^{ème} semaine, sous forme de diminution significative des capitons graisseux. Une réduction de 0,2 à 2 cm de tour de cuisse est constatée en fin de 4^{ème} semaine de traitement, et de 0,4 à 2,5 cm en fin de 8^{ème} semaine chez 60% des volontaires suivant le traitement.

### Exemple 2

Une huile drainante amincissante à base d'huile de chaulmoogra ayant la composition indiquée ci-après est préparée suivant les techniques usuelles :

| | | |
|---|---|---|
| huile de chaulmoogra | | 5,0 |
| extrait huileux de thé vert | | 5,0 |
| extrait huileux de café vert | | 5,0 |
| extrait huileux de Garcinia cambodgiana | | 5,0 |
| extrait huileux de Chondrus crispus | | 5,0 |
| huile de noisette | | 1,0 |
| huile de camélia | | 30,0 |
| isohexadécane | | 20,0 |
| parfum | | 1, 0 |
| triglycéride caprique / caprylique | qsp | 100,0 |

Les extraits huileux utilisés dans la composition ci-dessus sont obtenus dans un mélange de triglycérides caprique / caprylique.

### Exemple 3

Une crème amincissante à base d'huile de chaulmoogra ayant la composition indiquée ci-après est préparée suivant les techniques usuelles :

| | | |
|---|---|---|
| huile de chaulmoogra | | 3,0 |
| glycérine | | 2,0 |
| EDTA trisodique | | 0,1 |
| butylène glycol | | 4,0 |
| gomme xanthane | | 0,2 |
| cétéaryl glucoside | | 4,0 |
| alcool cétylique | | 1,0 |
| tristéarine | | 0,8 |
| stéarate d'acétyl glycol | | 0,8 |
| phényl diméthicone | | 1,0 |
| cyclopentasiloxane | | 4,0 |
| tocophérol | | 0,7 |
| copolymère acrylate de sodium / laurate de | | |
| diméthyl acryloyle | | 0,5 |
| isohexadécane | | 0,3 |
| Polysorbate 80 | | 0,8 |
| extrait glycolique de lierre | | 2,0 |
| théophylline | | 1,0 |
| conservateurs | | 0,8 |
| parfums | | 0,1 |
| Eau déminéralisée | q.s.p. | 100,0 |

### Exemple 4

Un gel amincissant à base d'association de caféine et d'huile de chaulmoogra ayant la composition indiquée ci-après est préparé suivant les techniques usuelles :

| | | |
|---|---|---|
| liposomes d'huile de chaulmoogra (correspondant | | |
| à 0,5% d'huile de chaulmoogra) | | 10,0 |
| liposomes de caféine (correspondant à 0,4% | | |
| de caféine) | | 10,0 |
| glycérine | | 4,0 |
| mannuronate de méthyl silanol | | 5,0 |
| polymère carboxyvinylique | | 1,0 |
| trométhamine | | 1,2 |
| eau distillée de cannelle | | 20,0 |
| eau distillée de bourgeons de sapins | | 5,0 |
| eau distillée de piment | | 1,0 |
| conservateur | | 0,1 |
| Eau déminéralisée | q.s.p. | 100,0 |

Ce gel amincissant est utilisé en application topique, une fois par jour sur les zones de la peau à traiter, pendant une période de 6 à 8 semaines. Les premiers effets peuvent être observés dès la fin de la 2^{ème} semaine. Les essais effectués sur un groupe de 20 volontaires ont permis d'observer une diminution significative des capitons graisseux. Une réduction de 0,2 à 2,5 cm de tour de cuisse est constatée en fin de 4^{ème} semaine de traitement, et de 0,5 à 3 cm en fin de 8^{ème} semaine chez 70% des volontaires suivant le traitement.

### Exemple 5

Une crème amincissante à base d'association de caféine et d'huile de chaulmoogra ayant la composition indiquée ci-après est préparée suivant les techniques usuelles :

| | | |
|---|---|---|
| huile de chaulmoogra | | 2,5 |
| caféine | | 2,0 |
| glycérine | | 2,0 |
| EDTA trisodique | | 0,1 |
| butylène glycol | | 4,0 |
| gomme xanthane | | 0,2 |
| cétéaryl glucoside | | 4,0 |
| alcool cétylique | | 1,0 |
| tristéarine | | 0,8 |
| stéarate d'acétyl glycol | | 0,8 |
| phényl diméthicone | | 1,0 |
| cyclopentasiloxane | | 4,0 |
| tocophérol | | 0,7 |
| copolymère acrylate de sodium / laurate de | | |
| diméthyl acryloyle | | 0,5 |
| isohexadécane | | 0,3 |
| Polysorbate 80 | | 0,8 |
| conservateurs | | 0,8 |
| parfums | | 0,1 |
| Eau déminéralisée | q.s.p. | 100,0 |

### Exemple 6

Un gel amincissant à base d'association de caféine, de guggulipides et d'huile de chaulmoogra ayant la composition indiquée ci-après est préparé suivant les techniques usuelles :

| | | |
|---|---|---|
| huile de chaulmoogra | | 1,0 |
| guggulipides | | 1,0 |
| caféine | | 0,5 |
| théophylline | | 0,2 |
| PEG 400 | | 2,0 |
| glycérine | | 2,0 |
| alcool éthylique à 96% | | 20,0 |
| polymère carboxyvinylique | | 0,5 |
| potasse | | 0,3 |
| octyldocécanol | | 1,0 |
| oléate de décyle | | 1,0 |
| ammonium acryloyl diméthyl laurate / | | |
| VP copolymère | | 0,7 |
| lauryl méthicone copolyol | | 0,5 |
| Tween 20 | | 0,5 |
| extrait glycolique de lierre | | 2,0 |
| parfums | | 0,1 |
| Eau déminéralisée | q.s.p. | 100,0 |

### Exemple 7

Une crème amincissante à base d'association de caféine et d'huile de chaulmoogra ayant la composition indiquée ci-après est préparée suivant les techniques usuelles :

| | | |
|---|---|---|
| huile de chaulmoogra | | 2,5 |
| huile de macadamia | | 2,0 |
| huile de noisette | | 3,0 |
| octyl dodécanol | | 3,0 |
| Tween 60 | | 3,5 |
| Span 60 | | 2,5 |
| alcool butylique | | 0,5 |
| alcool béhénylique | | 1,5 |
| Eau déminéralisée | q.s.p. | 100,0 |
| caféine | | 1,5 |
| théobromine | | 0,5 |
| EDTA trisodique | | 0,1 |
| Polymère carboxyvinylique | | 0,3 |
| éthoxy diglycol | | 5,0 |
| trométhamine | | 0,5 |
| glycérine | | 2,0 |
| escine | | 0,3 |
| alcool éthylique à 96° | | 5,0 |
| conservateurs | | 0,6 |
| parfums | | 0,2 |

## Revendications

1. Utilisation cosmétique de l'huile de chaulmoogra et/ou de ses composants, éventuellement en combinaison avec des guggulipides et/ou de la Z-guggulstérone et de la E-guggulstérone, pour le traitement et la prévention des surcharges adipeuses et de la cellulite.

2. Utilisation de l'huile de chaulmoogra et/ou de ses composants, éventuellement en combinaison avec des guggulipides et/ou de la Z-guggulstérone et de la E-guggulstérone, pour préparer une composition pharmaceutique destinée au traitement et à la prévention des surcharges adipeuses et de la cellulite.

3. Utilisation selon la revendication 1 ou 2, **caractérisée en ce que** les composants de l'huile de chaulmoogra sont les acides chaulmoogrique, hydnocarpique et gorlique, ainsi que leurs sels et esters.

4. Utilisation selon la revendication 3, **caractérisée en ce que** la composition comprend les sels ou esters des acides chaulmoogrique, hydnocarpique et gorlique.

5. Utilisation selon la revendication 4, **caractérisée en ce que** les sels et esters sont choisis parmi les esters de méthyle, d'éthyle ou de benzyle, ainsi que les sels de sodium ou de potassium.

6. Utilisation selon l'une quelconque des revendications précédentes, **caractérisée en ce que** la teneur en huile de chaulmoogra, ou en acides ou sels ou esters est comprise entre 0,1 et 20% en poids par rapport au poids total de la composition.

7. Utilisation selon la revendication 6, **caractérisée en ce que** la teneur en huile de chaulmoogra, ou en acides ou sels ou esters est comprise entre 1 et 10% en poids par rapport au poids total de la composition.

8. Utilisation selon la revendication 1 ou 2, **caractérisée en ce que** les guggulipides sont obtenus par extraction à l'acétate d'éthyle de la gomme-résine provenant de l'écorce de *Commiphora mukul,* séchée et broyée.

9. Utilisation selon la revendication 1, 2 ou 8, **caractérisée en ce que** la teneur en guggulipides est comprise entre 0,05 et 20% en poids par rapport au poids total de la composition.

10. Utilisation selon la revendication 9, **caractérisée en ce que** la teneur en guggulipides est comprise entre 1 et 10% en poids par rapport au poids total de la composition.

11. Composition cosmétique et/ou pharmaceutique destinée au traitement et à la prévention des surcharges adipeuses et de la cellulite, **caractérisée en ce qu'**elle comprend de l'huile de chaulmoogra et/ou un au moins de ses composants et éventuellement des guggulipides et/ou de la Z-guggulstérone et de la E-guggulstérone, en combinaison avec une ou plusieurs bases xanthiques.

12. Composition selon la revendication 11, **caractérisée en ce que** les composants de l'huile de chaulmoogra sont les acides chaulmoogrique, hydnocarpique et gorlique, ainsi que leurs sels et esters.

13. Composition selon la revendication 12, **caractérisée en ce que** les sels et esters sont choisis parmi les esters de méthyle, d'éthyle ou de benzyle, ainsi que les sels de sodium ou de potassium.

14. Composition selon l'une quelconque des revendications 11 à 13, **caractérisée en ce que** le rapport en poids des bases xanthiques à l'huile de chaulmoogra est compris entre 1:0,5 et 1:5.

15. Composition selon l'une quelconque des revendications 11 à 14, **caractérisée en ce que** la teneur en huile de chaulmoogra, ou en acides ou sels ou esters est comprise entre 0,1 et 15% en poids, et la teneur en base xanthique est comprise entre 0,1 et 8% en poids par rapport au poids total de la composition.

16. Composition selon la revendication 15, **caractérisée en ce que** la teneur en huile de chaulmoogra, ou en acides ou sels ou esters est comprise entre 1 et 8% en poids par rapport au poids total de la composition.

17. Composition selon l'une quelconque des revendications 11 à 16, **caractérisée en ce que** l'huile de chaulmoogra, ou ses acides ou esters, et/ou les guggulipides, et la base xanthique, sont sous forme de liposomes.

18. Composition selon l'une quelconque des revendications 11 à 17, **caractérisée en ce que** les bases xanthiques sont la caféine, la théophylline et la théobromine, isolément ou en association.

19. Composition selon la revendication 18, **caractérisée en ce que** la base xanthique est la caféine.

20. Composition selon l'une quelconque des revendications 11 à 19, **caractérisée en ce qu'**elle comprend en outre un agent veinotonique.

21. Composition selon la revendication 20, **caractérisée en ce que** l'agent veinotonique est choisi parmi les extraits végétaux à saponosides, les extraits végétaux à coumarine, et les extraits végétaux à flavonoïdes.

22. Composition selon l'une quelconque des revendications 11 à 21, **caractérisée en ce qu'**elle contient en outre un agent restructurant.

23. Procédé de traitement cosmétique de la peau, pour prévenir ou réduire les surcharges adipeuses et la cellulite, par application d'une composition à base d'huile de chaulmoogra et éventuellement de guggulipides et/ou de Z-guggulstérone et de E-guggulstérone sur la zone de la peau nécessitant un tel traitement.

## Claims

1. Cosmetic use of chaulmoogra oil and/or its components, optionally in association with guggulipids and/or Z-guggulsterone and E-guggulsterone, for treating and preventing adipose excess and cellulitis.

2. Use of chaulmoogra oil and/or its components, optionally in association with guggulipids and/or Z-guggulsterone and E-guggulsterone, in the preparation of a pharmaceutical composition for treating and preventing adipose excess and cellulitis.

3. Use according to claim 1 or 2, **characterized in that** the chaulmoogra oil components are chaulmoogric, hydnocarpic and gorlic acids, as well as salts and esters thereof.

4. Use according to claim 3, **characterized in that** the composition comprises the salts or esters of the chaulmoogric, hydnocarpic and gorlic acids.

5. Use according to claim 4, **characterized in that** the salts and esters are selected from the methyl, ethyl or benzyl esters, as well as the sodium or potassium salts.

6. Use according to any one of the preceding claims, **characterized in that** the amount of chaulmoogra oil, or of acids or salts or esters is comprised between 0,1 and 20% by weight of the total composition weight.

7. Use according to claim 6, **characterized in that** the amount of chaulmoogra oil, or of acids or salts or esters, is comprised between 1 and 10% by weight of the total composition weight.

8. Use according to claim 1 or 2, **characterized in that** the guggulipids are obtained by extraction with ethyl acetate of the resin-rubber coming from dried and ground *Commiphora mukul* bark.

9. Use according to claim 1, 2 or 8, **characterized in that** the amount of guggulipids is comprised between 0,05 and 20% by weight of the total composition weight.

10. Use according to claim 9, **characterized in that** the amount of guggulipids is comprised between 1 and 10% by weight of the total composition weight.

11. Cosmetic and/or pharmaceutical composition for treating and preventing adipose excess and cellulitis, **characterized in that** it comprises chaulmoogra oil and/or at least one of its components, and optionally guggulipids and/or Z-guggulsterone and E-guggulsterone, in association with one or several xanthic bases.

12. Composition according to claim 11, **characterized in that** the components of chaulmoogra oil are the chaulmoogric, hydnocarpic and gorlic acids, as well as salts and esters thereof.

13. Composition according to claim 12, **characterized in that** the salts or esters are selected from the methyl, ethyl or benzyl esters, as well as the sodium or potassium salts.

14. Composition according to any one of claims 11 to 13, **characterized in that** the xanthic bases : chaulmoogra oil weight ratio is comprised between 1:0,5 and 1:5.

15. Composition according to any one of claims 11 to 14, **characterized in that** the amount of chaulmoogra oil, or of acids or salts or esters, is comprised between 0,1 and 15% by weight, and the amount of xanthic base is comprised between 0,1 and 8% by weight of the total composition weight.

16. Composition according to claim 15, **characterized in that** the amount of chaulmoogra oil, or of acids or salts or esters is comprised between 1 and 8% by weight of the total composition weight.

17. Composition according to any one of claims 11 to 16, **characterized in that** the chaulmoogra oil, or its acids or esters, and/or the guggulipids, and the xanthic base, are in the form of liposome.

18. Composition according to any one of claims 11 to 17, **characterized in that** the xanthic bases are selected from caffeine, theophylline and theobromine, or one of their mixtures.

19. Composition according to claim 18, **characterized in that** the xanthic base is caffeine.

20. Composition according to any one of claims 11 to 19, **characterized in that** it further comprises a phlebotonic agent.

21. Composition according to claim 20, **characterized in that** the phlebotonic agent is selected from saponosides plant extracts, coumarin plant extracts, and flavonoids plant extracts.

22. Composition according to any one of claims 11 to 21, **characterized in that** it further comprises a restructuration agent.

23. Skin cosmetic treatment method, for preventing or reducing adipose excess and cellulitis, by applying a composition based on chaulmoogra oil and optionally guggulipids and/or of Z-guggulsterone and E-guggulsterone, on the skin area requiring such treatment.

## Patentansprüche

1. Kosmetische Verwendung von Chaulmoograöl und/oder Komponenten davon, gegebenenfalls in Kombination mit Guggulipiden und/oder Z-Guggulsteron und E-Guggulsteron, zur Behandlung und Prävention von adipösem Übergewicht und Cellulite.

2. Verwendung von Chaulmoograöl und/oder Komponenten davon, gegebenenfalls in Kombination mit Guggulipiden und/oder Z-Guggulsteron und E-Guggulsteron, zur Herstellung einer pharmazeutischen Zusammensetzung zur Behandlung und Prävention von adipösem Übergewicht und Cellulite.

3. Verwendung nach Anspruch 1 oder 2, **dadurch gekennzeichnet, dass** die Komponenten des Chaulmoograöls Chaulmoogra-, Hydnocarpus- und Gorlisäure sowie Salze und Ester davon sind.

4. Verwendung nach Anspruch 3, **dadurch gekennzeichnet, dass** die Zusammensetzung Salze oder Ester von Chaulmoogra-, Hydnocarpus- und Gorlisäure umfasst.

5. Verwendung nach Anspruch 4, **dadurch gekennzeichnet, dass** die Salze und Ester aus Methylestern, Ethylestern und Benzylestern bzw. aus Natrium- und Kaliumsalzen ausgewählt sind.

6. Verwendung nach einem der vorangegangenen Ansprüche, **dadurch gekennzeichnet, dass** der Gehalt an Chaulmoograöl oder an Säuren, Salzen oder Estern zwischen 0,1 und 20 Gew.-%, bezogen auf das Gesamtgewicht der Zusammensetzung, liegt.

7. Verwendung nach Anspruch 6, **dadurch gekennzeichnet, dass** der Gehalt an Chaulmoograöl oder an Säuren, Salzen oder Estern zwischen 1 und 10 Gew.-%, bezogen auf das Gesamtgewicht der Zusammensetzung, liegt.

8. Verwendung nach Anspruch 1 oder 2, **dadurch gekennzeichnet, dass** die Guggulipide durch Ethylacetatextraktion des Gummiharzes von getrockneter und zerkleinerter Rinde von Commiphora mukul erhalten werden.

9. Verwendung nach Anspruch 1, 2 oder 8, **dadurch gekennzeichnet, dass** der Gehalt an Guggulipiden zwischen 0,05 und 20 Gew.-%, bezogen auf das Gesamtgewicht der Zusammensetzung, liegt.

10. Verwendung nach Anspruch 9, **dadurch gekennzeichnet, dass** der Gehalt an Guggulipiden zwischen 1 und 10 Gew.-%, bezogen auf das Gesamtgewicht der Zusammensetzung, liegt.

11. Kosmetische und/oder pharmazeutische Zusammensetzung zur Behandlung und Prävention von adipösem Übergewicht und Cellulite, **dadurch gekennzeichnet, dass** sie Chaulmoograöl und/oder zumindest eine Komponente davon und gegebenenfalls Guggulipide und/oder Z-Guggulsteron und E-Guggulsteron in Kombination mit einer oder mehreren Xanthinbasen umfasst.

12. Zusammensetzung nach Anspruch 11, **dadurch gekennzeichnet, dass** die Komponenten des Chaulmoograöls Chaulmoogra-, Hydnocarpus- und Gorlisäure sowie Salze und Ester davon umfassen.

13. Verwendung nach Anspruch 4, **dadurch gekennzeichnet, dass** die Salze und Ester aus Methylestern, Ethylestern und Benzylestern bzw. aus Natrium- und Kaliumsalzen ausgewählt sind.

14. Zusammensetzung nach einem der Ansprüche 11 bis 13, **dadurch gekennzeichnet, dass** das Gewichtsverhältnis zwischen Xanthinbasen und Chaulmoograöl zwischen 1:0,5 und 1:5 liegt.

15. Zusammensetzung nach einem der Ansprüche 11 bis 14, **dadurch gekennzeichnet, dass** der Gehalt an Chaulmoograöl oder Säuren, Salzen oder Estern zwischen 0,1 und 15 Gew.-% und der Gehalt an Xanthinbasen zwischen 0,1 und 8 Gew.-%, jeweils bezogen auf das Gesamtgewicht der Zusammensetzung, liegt.

16. Zusammensetzung nach Anspruch 15, **dadurch gekennzeichnet, dass** der Gehalt an Chaulmoograöl oder Säuren, Salzen oder Estern zwischen 1 und 8 Gew.-%, bezogen auf das Gesamtgewicht der Zusammensetzung, liegt.

17. Zusammensetzung nach einem der Ansprüche 11 bis 16, **dadurch gekennzeichnet, dass** das Chaulmoograöl oder die Säuren oder Ester davon und/oder die Guggulipide und die Xanthinbase in Form von Liposomen vorliegen.

18. Zusammensetzung nach einem der Ansprüche 11 bis 17, **dadurch gekennzeichnet, dass** die Xanthinbasen Coffein, Theophyllin und Theobromin, einzeln oder in Kombination, sind.

19. Zusammensetzung nach Anspruch 18, **dadurch gekennzeichnet, dass** die Xanthinbase Coffein ist.

20. Zusammensetzung nach einem der Ansprüche 11 bis 19, **dadurch gekennzeichnet, dass** sie außerdem ein venentonisierendes Mittel umfasst.

21. Zusammensetzung nach Anspruch 20, **dadurch gekennzeichnet, dass** das venentonisierende Mittel aus pflanzlichen Saponosidextrakten, aus pflanzlichen Cumarinextrakten und pflanzlischen Flavonoidextrakten ausgewählt ist.

22. Zusammensetzung nach einem der Ansprüche 11 bis 21, **dadurch gekennzeichnet, dass** sie außerdem ein restrukturierendes Mittel enthält.

23. Verfahren zur kosmetischen Behandlung der Haut zur Prävention oder Reduktion von adipösem Übergewicht und Cellulite durch Auftragen einer Zusammensetzung auf der Basis von Chaulmoograöl und gegebenenfalls Guggulipiden und/oder Z-Guggulsteron und E-Guggulsteron auf jene Hautregion, die einer solchen Behandlung bedarf.
